# EUROPÄISCHE PATENTANMELDUNG

(11) **EP 0 554 675 A1**
(43) Veröffentlichungstag der Anmeldung: **11.08.1993**
(21) Anmeldenummer: 93100399.0
(22) Anmeldetag: 13.01.1993
(51) Int. Cl.: C08G 61/02, C07C 69/025, C07C 69/767

(54) **Verfahren zur Herstellung von Polymeren des para-Xylylens und von diesem abgeleiteten Derivaten**

(30) Priorität: 31.01.1992 DE 4202672
(71) Anmelder: HOECHST AKTIENGESELLSCHAFT, D-65926 Frankfurt am Main (DE)
(72) Erfinder: Greiner, Andreas, Dr., W-3550 Marburg (DE); Simon, Peter, W-3550 Marburg/Bauerbach (DE)

(57) **Zusammenfassung**

Die Erfindung betrifft ein Verfahren zur Herstellung von Verbindungen der Formel (I) 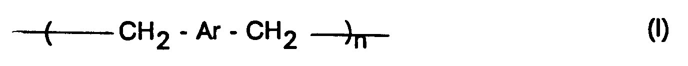
durch Pyrolyse, wobei
- n: der Polymerisationsgrad ≧ 2 ist.

Dabei pyrolisiert man die Verbindungen der Formel (II)

R-CO-O-CH₂-Ar-CH₂-O-CO-R (II)

einstufig unter Bedingungen, die zur Spaltung der Esterbindungen unter einem Partialdruck der Ester von p ≦ 10 mbar bei Temperaturen T ≧ 500° C zur Bildung der korrespondierenden Chinodimethanderivate führen, ohne daß diese jedoch in Folgereaktionen weiter gespalten werden. Anschließend wurde die durch spontane Polykondensation der Chinodimethanderivate entstandenen Polymeren des p-Xylylens abgekühlt, Ar und R haben folgende Bedeutungen:
- - Ar -: bedeutet einen 1,4-Phenylen oder einen anderen C₆-C₁₂ aromatischen oder heteroaromatischen Rest, bei dem die Bindungen in para oder vergleichbarer koaxialer oder antiparalleler Stellung stehen, welcher gegebenenfalls mit einem oder mehreren verzweigten oder unverzweigten, gegebenenfalls halogensubstituierten, C₁-C₄-Alkyl-, Alkenyl- oder Alkoxyresten oder C₆-C₁₂-Aryl oder Heteroarylresten, Hydroxy-, Nitro-, Nitril- oder Aminogruppen oder mit Resten R*-S- oder R*-CO-O, wobei R* für einen C₁-C₄-Alkyl- oder einen C₆-C₁₂-Arylrest steht, substituiert ist;
- R-: bedeutet einen verzweigten oder unverzweigten C₁-C₄-Alkyl- oder C₆-C₁₂-Arylrest oder einen Rest R*-CO-O-, wobei R* die vorstehend genannte Bedeutung hat.

## Beschreibung

Poly-para-xylylen ist ein Material mit außergewöhnlichen chemischen und physikalischen Eigenschaften. Es ist ein kristallines Polymer, welches bei Zimmertemperatur von keinem bekannten organischen Lösemittel angegriffen wird. Seine Schmelz- und Zersetzungstemperatur liegt bei 420° C. Im einzigen technisch realisierten Herstellungsverfahren (Encyclopedia of Polym. Sci. Tech. 15, 98, New York 1971) wird Poly-p-xylylen durch spontane Polymerisation von 1.4-Dimethylen-2.5-cyclohexadien hergestellt, welches aus der Dampfphase auf einer unter eine gewisse kritische Temperatur thermostatisierte Oberfläche kondensiert wird. Daher eignet sich Poly-para-xylylen besonders als chemisch, thermisch und mechanisch stabiler Überzug. Typische Anwendungen sind Beschichtungen von mikroelektronischen Bauteilen zum Ausschluß von Feuchtigkeit und Schmutz, Dielektra in Kondensatoren hoher Packungsdichte, Partikelenkapsulierung und Gas-Barriereschichten (Encyclopedia of Polym. Sci. Tech. 15, 98, New York 1971).

Die Herstellung von Poly-p-xylylen nach dem Stande der Technik geschieht in zwei aufeinanderfolgenden Pyrolyseschritten aus p-Xylol. Im ersten Schritt wird p-Xylol unter Zusatz von Wasserdampf, Methan oder Stickstoff bei 950° C zu Tricyclo-^{[8.2.2.2.4,7]-}hexadeca-4,6,10,12,13,15-hexaen (Paracyclophan) pyrolisiert (US-A-3 149 175, US-A-3 412 167). Im folgenden zweiten Schritt wird Paracylcophan zu 1.4.-Dimethylen-2.5-cyclohexadien (Chinodimethan) gespalten und dieses unterhalb einer kritischen Temperatur auf einer Oberfläche kondensiert, wo es spontan polymerisiert. Diese Pyrolyse wird bei Temperaturen von 550 bis 700° C und Drücken von ≦ 1.5 mbar geführt (US-A-3 342 754, US-A-3 288 728, US-A-3 300 322).

Der Nachteil des Prozesses besteht vor allem darin, daß zwei Pyrolysestufen erforderlich sind. Das eigentlich als Monomerbaustein zu betrachtende Paracyclophan kann nur sehr aufwendig und mit mäßigen Ausbeuten hergestellt werden. Alternative Darstellungsmethoden von Paracyclophan (EP-A-294 651) oder des Polymeren (GB-A-807 196) via Hoffmann-Eliminierung bieten wegen der hohen entstehenden Salzfracht keine Vorteile für technische Verfahren.

Ebenfalls nachteilig ist, daß substituierte Poly-p-xylylene nicht durch Einsatz der käuflichen substituierten p-Xylole gewonnen werden können. Die Substituenten müssen vielmehr durch präparative organisch-chemische Methoden auf der Stufe des Paracyclophans eingeführt werden (US-A-3 288 728). Alternative Darstellungswege substituierter Paracyclophane sind mit erheblichem präparativem Aufwand verknüpft.

Versuche, Poly-p-xylylen durch direkte einstufige Pyrolyse von Xylol bei bis zu 1000° C herzustellen, waren wenig erfolgreich. Es entstanden schlecht definierbare, vernetzte Polymere mit deutlicher Färbung (GB-A-650 947, GB-A-673 651).

Es besteht daher die Aufgabe, einen einfachen und wirtschaftlichen Prozeß für die Herstellung von Poly-p-xylylen und verwandten Derivaten zu entwickeln.

Gelöst wird diese Aufgabe erfindungsgemäß durch ein Verfahren, in dem leicht zugängliche Monomere in einer Stufe zum Polymer pyrolisiert werden.

Die vorliegende Anmeldung betrifft daher ein Verfahren zur Herstellung von Verbindungen der Formel (I) 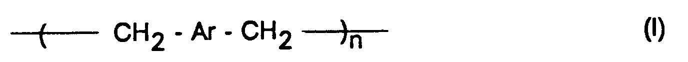
Hierbei werden Verbindungen der Formel (II)

R-CO-O-CH₂-Ar-CH₂-O-CO-R (II)

einstufig pyrolisiert unter Bedingungen, die zur Spaltung der Esterbindungen unter einem Partialdruck der Ester von p ≦ 10 mbar bei Temperaturen T ≧ 500° C unter Bildung der korrespondierenden Chinodimethanderivate führen, ohne daß diese jedoch in Folgereaktionen weiter gespalten werden und anschließender Abkühlung und spontaner Polymerisation unter Kondensation der Chinodimethanderivate, wobei Ar, R und n folgende Bedeutung haben:
- - Ar -: bedeutet einen 1,4-Phenylen oder einen anderen C₆-C₁₂ aromatischen oder heteroaromatischen Rest, bei dem die Bindungen in para oder vergleichbarer koaxialer oder antiparalleler Stellung stehen, welcher gegebenenfalls mit einem oder mehreren verzweigten oder unverzweigten, gegebenenfalls halogensubstituierten, C₁-C₄-Alkyl-, Alkenyl- oder Alkoxyresten oder C₆-C₁₂-Aryl- oder Heteroarylresten, Hydroxy-, Nitro-, Nitril- oder Aminogruppen oder mit Resten R*-S- oder R*-CO-O, wobei R* für einen C₁-C₄-Alkyl- oder einen C₆-C₁₂-Arylrest steht, substituiert ist;
- R-: bedeutet einen verzweigten oder unverzweigten C₁-C₄-Alkyl- oder C₆-C₁₂-Arylrest oder einen Rest R*-CO-O-, wobei R* die vorstehend genannte Bedeutung hat und
- n: der Polymerisationsgrad ≧ 2 ist.

Als Ausgangssubstanzen werden erfindungsgemäß Verbindungen der Formel

HOCH₂-Ar-CH₂OH

eingesetzt.

Die Xylylendiole werden nach bekannten Methoden der organischen Chemie mit Carbonsäurechloriden R-COCl zu den Xylylendiestern R-CO-OCH₂-Ar-CH₂O-CO-R umgesetzt, wobei R einen niederen C₁-C₄-Alkylrest im Fall eines monofunktionellen Säurechlorids bedeutet, aber auch eine weitere Säurechloridfunktion -COCl darstellen kann. Als geeignete mono- bzw. difunktionelle Reagenzien haben sich Acetylchlorid und Oxalsäuredichlorid erwiesen. Sofern zur Acetylierung der Xylylendiole bifunktionelle Säurederivate eingesetzt werden, werden die entstehenden Esterchloride Cl-C₂O₃-CH₂-Ar-CH₂-C₂O₃-Cl mit Alkoholen R*-OH umgesetzt zu den Estern R*-C₂O₄-CH₂-Ar-CH₂-C₂O₄-R*, wobei R* einen verzweigten oder unverzweigten C₁-C₄ Alkylrest oder einen C₆-C₁₂ Arylrest bedeutet.
Erfindungsgemäß werden die so erhaltenen Ester einer Pyrolyse unter vermindertem Druck bei Temperaturen von 500° C bis 1000° C, bevorzugt von 700 und 900° C unterzogen. Die Reaktionsdrücke betragen p ≦ 10 mbar, vorzugsweise p ≦ 1 mbar.

Die Verweilzeiten in der Pyrolysezone werden so gewählt, daß die Ester möglichst weitgehend zu den korrespondierenden Chinodimethanen zersetzt werden, ohne daß diese jedoch in Folgereaktionen weiter thermisch gespalten werden. Die gasfömigen Chinodimethane werden abgekühlt und auf einer thermostatisierten Oberfläche auskondensiert, wo sie spontan polymerisieren. Die Temperatur der Kondensationsoberfläche liegt unter einer kritischen Kondensations-/Polymerisationstemperatur, oberhalb welcher keine Kondensation und Polymerisation der Chinodimethane stattfindet. Für die Darstellung des unsubstituierten Poly-para-xylylens liegt diese Temperatur bei 30° C, für substituierte Poly-para-xylylene liegen die Temperaturen höher, jedoch nicht über 130° C. Bei der in der Kondensationszone herrschenden Temperatur ist der Sättigungsdampfdruck der Chinodimethane kleiner als der Systemdruck.

Man erhält Poly-p-xylylen als dünnen, farblosen Film auf der Kondensationsfläche. Es ist in siedendem Chlornaphthalin oder Benzylbenzoat löslich, aus dem es beim Abkühlen wieder ausfällt. Das erfindungsgemäß hergestellte unsubstituierte Poly-p-xylylen erweicht bei Temperaturen von T ≧ 300° C. Es entspricht folglich den in der US-A-3 342 754 angegebenen Spezifikationen für lineares, unvernetztes Poly-p-xylylen, im Gegensatz zu den Produkten, die aus der einstufigen Hochtemperaturpyrolyse von p-Xylol (GB-A-650 947, GB-A-673 651) entstehen.

Ein besonderer Vorteil des erfindungsgemäßen Verfahrens liegt darin, daß Substituenten am aromatischen Ring sehr einfach und in wohldefinierter Weise eingeführt werden können. So können zur Darstellung substituierter Xylylendiester die käuflichen oder durch bekannte Umsetzungen der Organischen Chemie leicht erhältlichen substituierten Xylylendiole eingesetzt werden oder es können alternativ die Substituenten am aromatischen Ring durch analoge Umsetzungen mit den Xylylendiestern eingeführt werden. Die Pyrolyse kann dann ganz analog zur Pyrolyse der unsubstituierten Xylylendiester durchgeführt werden.

Nach dem Stand der Technik dagegen werden Substituenten auf der Stufe des Paracyclophans eingeführt, wobei man schwer zu trennende Isomerengemische erhält. Die substituierten Homopolymere erhält man nach dieser Methode durch fraktionierende Kondensation, indem die unterschiedlich substituierten Chinodimethanderivate bei verschiedenen Temperaturen kondensiert und polymerisiert werden. Durch nicht fraktionierende Kondensation bei Zimmertemperatur erhält man nach dem bekannten Stand der Technik dagegen statistische Copolymere der unterschiedlich substituierten Chinodimethane (US-A-3 288 728).

Das hier beschriebene Verfahren gestattet ebenfalls auf sehr einfache Weise die Darstellung statistischer Copolymere. Zu diesem Zweck wird bei dem Pyrolyseschritt ein wohldefiniertes Gemisch zweier oder mehrerer unterschiedlich substituierter Xylylendiester eingesetzt und die Komponenten der Mischung gemeinsam bei identischen Bedingungen pyrolisiert. Das Gemisch der entstehenden substituierten Chinodimethane wird gemeinsam bei einer Temperatur unterhalb der kritischen Kondensationstemperatur des leichtestflüchtigen Derivats auskondensiert und liefert ein statistisches Copolymer.

### Beispiele:

### Beispiel 1:

### Darstellung von 1.4-Bis (isopropoxyoxalatomethyl)-benzol

32 ml 1.4-Bis-(hydroxymethyl)-benzol wurden unter Luft - und Feuchtigkeitsausschluß in 150 ml Toluol und 50 ml THF gelöst. Bei 0° C wurden nun 10 g Oxalsäuredichorid gelöst in 150 ml wasserfreiem THF und 50 ml wasserfreiem Toluol zugetropft, die Mischung zunächst bei Zimmertemperatur und dann unter Rückfluß gerührt und schließlich die Lösemittel und überschüssiges Säurechlorid im Vakuum abdestilliert. Das als fester Rückstand in 88 % der theoretischen Ausbeute verbleibende 1.4-Bis(chloro-oxalatomethyl)-benzol wurde im Vakuum getrocknet. 4,54 g der Substanz wurden unter Luft- und Feuchtigkeitsausschluß in Toluol gelöst. Durch Eintropfen dieser Lösung in 500 ml Isopropanol wurde 1.4-Bis-(isopropoxy-oxalatomethyl)-benzol C₆H₄(CH₂-C₂O₄C₃H₇)₂ als farbloser Niederschlag in 65 % Ausbeute erhalten.

### Beispiel 2:

### Darstellung von 1.4-Bis(acetatomethyl)-benzol

10,95 g 1.4-Bis(hydroxymethyl)-benzol wurden unter Luft- und Feuchtigkeitsausschluß in einem Kolben vorgelegt und 50 ml Acetylchlorid wurden zugetropft. Die Reaktionsmischung wurde bei Zimmertemperatur gerührt und das überschüssige Acetylchlorid abdestilliert. 1.4-Bis(acetatomethyl)-benzol wurde als schwach gelber Rückstand in 94 % der theoretischen Ausbeute erhalten.

### Beispiel 3:

### Pyrolyse der Ester

1 mmol der gemäß Beispiel 1 und 2 dargestellten Verbindungen wurde in ein Quarzschiffchen gefüllt und in einem Quarzrohr an den Anfang der Pyrolysezone in einem vorgeheizten Ofen (Heraeus Rohrofen RO 4/25 mit Regelgerät RE 1.1) plaziert. Nach der Evakuierung des Quarzrohres verdampfte die Substanz, strömte unter Zersetzung durch die Pyrolysezone und schlug sich als Film auf der Kondensationsfläche hinter dem Ofen nieder. Man erhielt Poly-p-xylylen bei Pyrolysetemperaturen oberhalb von 600°C. Die besten Ausbeuten ergaben sich bei Temperaturen von 700-900°C.

Die Elementaranalysen aller Polymerisationsprodukte stimmten innerhalb der experimentellen Fehlergrenzen mit den berechneten Werten für die Bruttozusammensetzung C₈H₈ überein. Die IR-Spektren der Folien entsprechen den für Poly-p-xylylen in der Literatur (Encyclopedia of Polym. Sci. Techn. 15, 98, New York 1971) angegebenen.

### Beispiel 4:

Darstellung von 1,4-Bis(pivalatomethyl)-2-chloro-benzol 3 g 1,4-Bis(hydroxymethyl)-2-chloro-benzol wurden unter Luft- und Feuchtigkeitsausschluß in einem Kolben vorgelegt und 20 ml Pivalinsäurechlorid zugetropft. Die Reaktionsmischung wurde bei Zimmertemperatur gerührt und das überschüssige Pivalinsäurechlorid abdestilliert. 1,4-Bis-(pivalatomethyl)-2-chloro-benzol wurde als blaßgelbe Flüssigkeit in 93 % der theoretischen Ausbeute erhalten.

### Beispiel 5:

Pyrolyse von 1,4-Bis-(pivalatomethyl)-2-chloro-benzol 1 mmol 1,4-Bis-(pivalatomethyl)-2-chloro-benzol wurde in ein Quarzschiffchen gefüllt und in einem Quarzrohr an dem Anfang der Pyrolysezone in einem vorgeheizten Ofen (Hereaus Rohrofen (RO 4/25 mit Regelgerät RE 1.1) plaziert. Nach Evakuierung des Quarzrohres verdampfte die Substanz, strömte unter Zersetzung durch die Pyrolysezone und schlug sich als Film auf der Kondensationsfläche hinter dem Ofen nieder. Man erhielt Poly-p-(chlorxylylen) bei Pyrolysetemperaturen oberhalb 700°C. Die besten Ausbeuten ergaben sich bei Temperaturen von 800 - 900°C.

## Patentansprüche

1. Verfahren zur Herstellung von Polymeren des p-Xylylens der Formel (I) 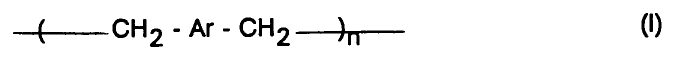 durch Pyrolyse, wobei
n der Polymerisationsgrad ≧ 2 ist,
dadurch gekennzeichnet, daß man Esterverbindungen der Formel (II)
R-CO-O-CH₂-Ar-CH₂-O-CO-R (II)
einstufig unter Bedingungen pyrolisiert, die zur Spaltung der Esterbindungen unter einem Partialdruck der Ester von p ≦ 10 mbar bei Temperaturen T ≧ 500° C zur Bildung der korrespondierenden Chinodimethanderivate führen, ohne daß diese jedoch in Folgereaktionen weiter gespalten werden, und daß anschließend die durch spontane Polykondensation der Chinodimethanderivate entstandenen Polymeren des p-Xylylens abgekühlt werden, wobei Ar und R folgende Bedeutung haben:
- Ar - bedeutet einen 1,4-Phenylen oder einen anderen C₆-C₁₂ aromatischen oder heteroaromatischen Rest, bei dem die Bindungen in para oder vergleichbarer koaxialer oder antiparalleler Stellung stehen, welcher gegebenenfalls mit einem oder mehreren verzweigten oder unverzweigten, gegebenenfalls halogensubstituierten, C₁-C₄-Alkyl-, Alkenyl- oder Alkoxyresten oder C₆-C₁₂-Aryl oder Heteroarylresten, Hydroxy-, Nitro-, Nitril- oder Aminogruppen oder mit Resten R*-S- oder R*-CO-O, wobei R* für einen C₁-C₄-Alkyl- oder einen C₆-C₁₂-Arylrest steht, substituiert ist;
R- bedeutet einen verzweigten oder unverzweigten C₁-C₄-Alkyl- oder C₆-C₁₂-Arylrest oder einen Rest R*-CO-O-, wobei R* die vorstehend genannte Bedeutung hat.

2. Verfahren nach Anspruch 1, dadurch gekennzeichnet, daß die Pyrolysetemperatur im Bereich von 500 bis 1000° C, vorzugsweise im Bereich von 700 bis 900° C, liegt.

3. Verfahren nach Anspruch 1, dadurch gekennzeichnet, daß die Pyrolyse der Verbindungen der Formel (II) und die Kondensation der daraus entstandenen Chinodimethanderivate bei einem Partialdruck p ≦ 10 mbar, bevorzugt bei p ≦ 1 mbar, durchgeführt wird.

4. Verfahren nach Anspruch 1, dadurch gekennzeichnet, daß bei dem Pyrolyseschritt zwei oder mehrere unterschiedlich substituierte Xylylendiester eingesetzt werden.
